# DEMANDE DE BREVET EUROPEEN

(11) **EP 1 411 458 A1**
(43) Date de publication de la demande: **21.04.2004**
(21) Numéro de dépôt: 03291329.5
(22) Date de dépôt: 04.06.2003
(51) Int. Cl.: G06F 19/00, H04L 9/32

(54) **Dispositif d'assistance, de contrôle, de validation et de sécurisation de la prise de médicaments**

(30) Priorité: 21.06.2002 FR 0207690
(71) Demandeur: Spiess, Jean, 91370 Verrières le Buisson (FR)
(72) Inventeur: Spiess, Jean, 91370 Verrières le Buisson (FR)

(57) **Abrégé**

Dispositif permettant l'assistance, le contrôle, la validation et la sécurisation de la prise des médicaments.

L'invention concerne un dispositif basé sur l'utilisation d'un mobile téléphonique (C) programmable encore appelé radiotéléphone qui, grâce à la fonction "réveil" en mode éteint ou allumé, déclenche une sonnerie ou un vibreur avec affichage du nom du médicament, de son image et de sa posologie;

Grâce à l'utilisation de certaines touches, il est possible d'activer le début de la prise des médicaments, et éventuellement d'informer le médecin d'effets secondaires, information qui sera automatiquement transférée sur son P.C (A);

Les informations concernant la posologie te la nature de la prescription sont envoyés par le P.C (A) du docteur, ou par des serveurs centraux (B) et (F).

L'ensemble des transactions est doublement sécurisé par l'utilisation combinée du numéro de téléphone et du numéro de la carte Vitale du patient.

## Description

La présente invention concerne un dispositif pour:
- alerter l'usager de la nécessité de la prise d'un ou plusieurs médicaments suivant ordonnance du médecin et à prendre à des moments précis de la journée et sur une période déterminée
- l'informer du nom du produit à prendre, éventuellement d'indiquer des caractéristiques précises telles qu'une contre indication, ou la façon de prendre le médicament avant ,pendant ou après le repas... etc. et visualiser son image pour éviter de se tromper de produit.
- valider le droit de recevoir l'information confidentielle a savoir la prescription médicale.
- De valider la compatibilité des prises de médicament notamment en cas de prescriptions multiples
- Eventuellement de contrôler la prise des médicaments prescrits.
- Eventuellement alerter le médecin traitant d'effets secondaires indésirables.
- Eventuellement d'alerter le patient de rendez vous avec le corps médical en général.
- Valider que le patient accepte la circulation de l'information sur les réseaux Télécom.

Jusqu'à présent, le contrôle de prise des médicaments était essentiellement assuré par des systèmes mécaniques de type boîtiers ou informatiques.

Le dispositif selon l'invention est principalement basé sur l'utilisation des réseaux téléphoniques et les téléphones mobiles de nouvelle génération permettant le développement d'applications locales par programmation ce qui autorise la mise en oeuvre des fonctions suivantes:
- Réception de données informatiques en provenance d'un fichier médical et véhiculé par un réseau Télécom comme par exemple mais de façon non limitative, le réseau Internet.
- Traitement en local de la prescription du médecin en transformant des données brut en horaires à respecter. le début de la prise de médicaments peut être défini comme étant celui de la réception validé de la prescription ou celui correspondant à l'appui d'une touche de validation de type "yes" sur le mobile.
- Contrôle et autorisation du récipiendaire par fourniture du code de la carte Vitale.
- Déclenchement d'une sonnerie ou d'un vibreur du mobile avec affichage sur l'écran, du nom du médicament, d'une image de celui ci et éventuellement de modalités liées à la prise elle même.
- Eventuellement, en cas de prescriptions multiples, par un ou plusieurs médecins, vérification automatique de compatibilité par envoi de la liste des médicaments à prendre à un centre de contrôle (B) utilisant les données des laboratoires de produits pharmaceutiques.

Un tel procédé permet non seulement de minimiser les temps de connexion au réseau Télécom grâce au traitement local dans le mobile, mais également d'assurer des contrôles multiples grâce à des échanges sécurisés d'informations en parties confidentielles, le tout grâce à un objet courant tel que le téléphone mobile dont l'usage se généralise rapidement. A noter que la fonction réveil du téléphone fonctionne aussi bien en mode éteint ou allumé ; il est donc aisé de programmer des heures de prises de médicament et de déclencher l'alarme que le mobile soit allumé ou éteint.

Par extension, le procédé peut s'appliquer à des piqûres, comme par exemple pour les diabétiques ou à des manipulations comme par exemple mais de façon non limitative les kinésithérapeutes à des heures prédéterminées.

Egalement par extension, le dispositif peut gérer des prises de rendez vous fixé à l'avance grâce à un rappel automatique; la prise de rendez vous peut être partie intégrante de l'information communiquée en consultation et dans ce cas faire partie d'un complément d'information de la prescription elle même, ou alors faire l'objet par exemple mais de façon non limitative d'un envoi de type SMS.

La réception de l'information tel qu'un rendez vous ou une prescription peut se faire de trois manières différentes:
- Réception par exemple mais de façon non limitative d'un SMS ( short message service) ou MMS ( multimédia services); celui ci est envoyé automatiquement au moment de la consultation; une variante consiste à ce que l'information soit rapatriée ultérieurement par activation des données du cabinet médical par le mobile du patient.
- Réception ,au travers d'un fichier centralisé si l'organisme de règlement des actes médicaux crée une base de donnée nationale.
- Enregistrement des données par le patient sur son ordinateur personnel et transfert des données par exemple mais de façon non limitative par émail ou SMS ou MMS; un transfert grâce à ces standards de traitement et de transport permet une activation directe du terminal.

Le procédé utilisé par le dispositif, objet de l'invention comporte quatre phases distinctes à savoir:
- la saisie des données, l'envoi au téléphone mobile et l'enregistrement des données.
- le contrôle de la compatibilité des médicaments et la visualisation de l'image ainsi que d'éventuels commentaires associés à la prise.
- l'alarme et le contrôle de la prise du médicament.
- la remontée éventuelle d'information sur les effets secondaires vers le docteur ou le prescripteur du médicament.

La saisie des données(voir figure) peut s'effectuer par un PC (A) du médecin ,de toute institution médicale ou du patient lui même; un logiciel spécifique enverra ces données par exemple mais de manière non limitative par SMS ou MMS ou tout autre standard de communication avec les mobiles sous réserve que le patient ai accepté de donner son code de carte Vitale (E) ainsi que son numéro de téléphone mobile; ses deux données seront indissociables et permettrons une double sécurité: seul le téléphone mobile du patient recevra cette information; en outre pour activer l'information il faudra communiquer une fois sur le mobile son code de carte Vitale qui sera comparé à celui donné au moment de la prescription; l'activation ne se fera que si les deux informations sont identiques.

Cette saisie de donnée peut également être envoyée à un système central (F) de type, mais de façon non exclusive, "Sécurité Sociale" afin de faciliter en outre le règlement de la prescription; suivant la réglementation en vigueur, l'information peut être envoyée par ce système central à l'usager, après un contrôle de pertinence à définir.

L'envoi de l'information au mobile pourra se faire par tout standard de traitement des données permettant la lecture directe sur le mobile comme par exemple mais de façon non limitative les SMS ou MMS.

L'enregistrement comportera des données classiques d'une prescription comme par exemple le nombre de comprimés par jour, l'horaire des prises et une durée du traitement.

A ce stade, le programme de relance utilisant la fonction "réveil" du mobile peut être activé mais le décompte de la durée du traitement ne démarrera que lorsque le patient aura acheté ses médicaments. Il lui suffira d'appuyer pour la premier fois sur la touche d'acceptation de type "yes" ou "validation" pour démarrer le traitement; ensuite à chaque prise il devra arrêter la fonction "réveil" en appuyant sur cette touche de validation ;cet appui validera la prise et permettra de décompter le nombre de prises.

L'activation des données reçues, consiste à transformer des données médicales de type nombre , heures et durée, en horaires fixes d'activation de la fonction "réveil" du mobile grâce à un logiciel spécifique chargé préalablement sur le téléphone mobile.

L'activation de la fonction "réveil" s'accompagnera d'un affichage simultané du nom du médicament, de son image et éventuellement des recommandations particulières liées à la prise. Ces données seront communiquées par un serveur central(B) qui représente un fichier national ou même international alimenté par l'industrie pharmaceutique; ce même serveur fournira les éventuelles incompatibilités et activera une fonction spécifique comme par exemple mais de façon non limitative un clignotement et affichage des médicaments incompatibles.

En cas de prise simultané multiple, soit l'écran est assez grand pour afficher l'ensemble des données, soit il faudra autant d'affichages et donc de validation que de médicaments.

Dès l'activation de la première prise ,grâce à l'appui sur la touche "yes" ou "validation" par exemple ,le programme spécifique calculera automatiquement la fin du traitement.

Au cas où le médecin ait fixé un nouveau rendez vous lors de la consultation, l'information sera traitée comme la prescription et l'alarme du mobile se déclenchera suivant des modalités à préciser.

La remontée vers le docteur d'éventuels d'effets secondaires s'effectue simplement par appui sur une touche de type "no" ou "cancel" ce qui aura pour but d'envoyer un message précodé avec le nom du médicament incriminé.

L'appui de cette touche peut d'ailleurs générer une liste type d'effets indésirables.

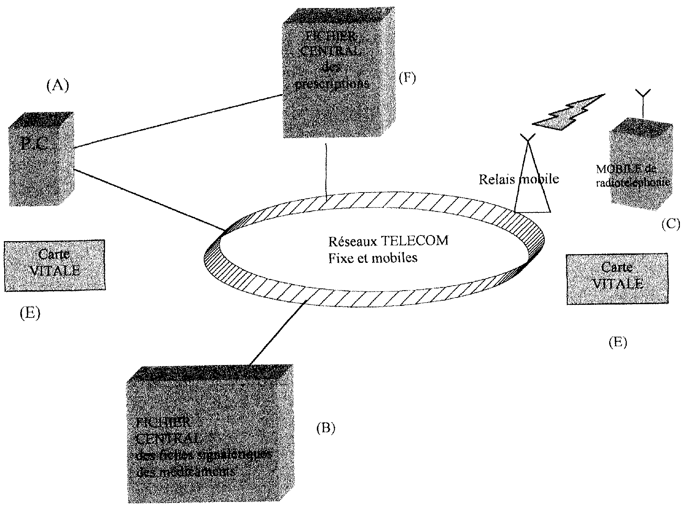

## Revendications

1. Dispositif de contrôle , de validation et de sécurisation de la prise de médicaments, **caractérisé par** la transmission des éléments d'une prescription à un téléphone mobile (C), laquelle transmission est sécurisée grâce à l'utilisation simultanée du numéro de téléphone mobile, ainsi que le code de la carte vitale (E) qui est fourni à l'émission et vérifié à la réception.

2. Dispositif selon la revendication 1, **caractérisé en ce que** la validation des compatibilités des médicaments est obtenue grâce à l'échange de données entre le mobile (C) et un fichier central(B) qui possède notamment les données relatives aux incompatibilités ainsi que les images des médicaments.

3. Dispositif selon l'une des revendications 1 à 2 **caractérisé en ce qu'**il contrôle la prise de médicament par appui sur une touche de type "validation" et d'alerter le patient grâce à l'utilisation de la fonction "réveil" des téléphones mobiles ou tout autre assistant personnel.

4. Dispositif selon l'une des revendications 1 à 3, **caractérisé en ce qu'**il informe automatiquement le médecin traitant des effets secondaires éventuels par appui d'une touche pré-codifiée comme par exemple "no" ,lors de la prise prévue, ce qui a pour effet d'envoyer une information de type mais non exclusivement SMS ou MMS , avec au moins le nom du produit, le nom du patient et éventuellement un type d'effet.

## Revendications modifiées

### Revendications modifiées conformément à la règle 86(2) CBE.

**1.** Dispositif décentralisé et autonome de contrôle, de validation et de sécurisation de la prise de médicaments, **caractérisé par** la transmission à un téléphone mobile (C), d'un programme encapsulé permettant de traiter les éléments d'une prescription médicale avec pour conséquence l'activation de la fonction réveil (du mobile) à chaque moment où une prise de médicament est nécessaire.

**2.** dispositif selon la revendication 1, **caractérisé par** son fonctionnement en mode allumé ou éteint du mobile téléphonique.

**3.** dispositif selon la revendication let 2, permettant la confirmation de la prise du médicament par simple appui sur une touche prédéterminée, qui arrête en même temps la fonction réveil.

**4.** dispositif selon les revendications1 à 3, **caractérisé par** une facturation simplifiée par les opérateurs Télécoms grâce à l'utilisation possible mais non exclusive des SMS+ ou MMS+

**5.** dispositif selon l'une des revendications 1 à 4, dans lequel,la transmission des données médicales est sécurisée grâce à l'utilisation simultanée du numéro de téléphone mobile, ainsi que le code de la carte vitale (E) qui est fourni à l'émission et vérifié à la réception.

**6.** Dispositif selon l'une des revendications 1 à 3, **caractérisé en ce qu'**il informe automatiquement le médecin traitant des effets secondaires éventuels par appui d'une touche pré codifiée comme par exemple "no", lors de la prise prévue, ce qui a pour effet d'envoyer une information de type mais non exclusivement SMS ou MMS, avec au moins le nom du produit, le nom du patient et éventuellement un type d'effet.
